# EUROPEAN PATENT APPLICATION

(11) **EP 4 645 201 A1**
(43) Date of publication of application: **05.11.2025**
(21) Application number: 23912223.7
(22) Date of filing: 27.12.2023
(51) Int. Cl.: G06Q 50/04, C12M 1/00, C12M 1/34, C12Q 1/02, G05B 19/418

(54) **CRITICAL PROCESS PARAMETER ANALYSIS METHOD, CRITICAL PROCESS PARAMETER ANALYSIS DEVICE, AND CRITICAL PROCESS PARAMETER ANALYSIS PROGRAM**

(30) Priority: 27.12.2022 JP 2022210765
(71) Applicant: The University of Osaka, Suita-shi, Osaka 565-0871 (JP)
(72) Inventor: KINOOKA, Masahiro, Suita-shi, Osaka 565-0871 (JP); SAITO, Atsuhiro, Suita-shi, Osaka 565-0871 (JP); MIZUTANI, Manabu, Suita-shi, Osaka 565-0871 (JP); OGAWA, Yuuki, Suita-shi, Osaka 565-0871 (JP)
(74) Representative: Meissner Bolte Partnerschaft mbB
(86) International application number: PCT/JP2023/046861
(87) International publication number: WO 2024/143445

(57) **Abstract**

An object is to specify a critical process parameter candidate in a manufacturing process for manufacturing a product. A critical process parameter analysis method of an aspect of this invention includes: a variation evaluation step (S2,S3) of evaluating, for each of motions in a manufacturing process of a product, (a) a degree of variation in a system input being input into a subject system from an outside of the subject system according to a motion parameter set to execute each motion and (b) a degree of variation in a system output being output from the subject system in response to each motion, the subject system being a subject of the motions; and a critical process parameter candidate specifying step (S4) of specifying a critical process parameter candidate based on a result of evaluating, for each motion, the degrees of the variations in the system input and in the system output.

## Description

### Technical Field

The present invention relates to a critical process parameter (CPP, important process parameter) analysis method and the like each specifying, in a manufacturing process of a product, a critical process parameter candidate which is highly likely to affect an important index reflecting an important quality characteristic of the product.

### Background Art

In order to set a manufacturing process which is capable of constantly supplying products having an intended function, a critical process parameter which affects an important quality characteristic of the product has been carried out. For example, the technique disclosed in Patent Literature 1 indicates a method for manufacturing a medicine product by using the Quality by Design approach. According to the Quality by Design approach, a target product quality profile and an important quality characteristic of an end product are set, a critical process parameter affecting a performance and a quality of the end product are specified on the basis of a risk assessment and a multivariate experiment, and ranges of an important quality characteristic and a critical process parameter necessary to ensure a target product quality profile are defined.

### Citation List

### [Patent Literature]

[Patent Literature 1]
International Publication No. WO 2019-044559

### Summary of Invention

### Technical Problem

Even in a case where motions in respective processes for manufacturing a product are carried out by, e.g., a device (machine), it might be impossible to completely control an input value of an input given by the device, whereby a fluctuation in the input value might occur. In a case where such a fluctuation exists, that is, the input value is unstable, this may possibly affect an end product. However, the technique disclosed in Patent Literature 1 does not take into consideration the fluctuation.

Further, in manufacturing of a cell preparation, a polymer, or the like, an outlier a cause of which is difficult to be specified may occur in a property of a subject after reaction. Thus, it is necessary to extract the critical process parameter while taking into consideration the instability in the property of the subject after reaction.

An aspect of the present invention has an object to provide a critical process parameter analysis method and a critical process parameter analysis device each of which specifies a critical process parameter candidate in a manufacturing process for manufacturing a product.

### Solution to Problem

In order to attain the above object, a critical process parameter analysis method in accordance with an aspect of the present invention includes: a variation evaluation step of evaluating, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and a critical process parameter candidate specifying step of specifying a critical process parameter candidate from among the motion parameters on a basis of a result of evaluating, for the each of the plurality of motions, the degree of the variation in the system input and the degree of the variation in the system output.

In order to attain the above object, a critical process parameter analysis device in accordance with an aspect of the present invention includes: a variation evaluation section that evaluates, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and a critical process parameter candidate specifying section that specifies a critical process parameter candidate from among the motion parameters on a basis of a result of evaluating, for the each of the plurality of motions, the degree of the variation in the system input and the degree of the variation in the system output.

### Advantageous Effects of Invention

In accordance with an aspect of the present invention, it is possible to specify a critical process parameter candidate in a manufacturing process for manufacturing a product.

### Brief Description of Drawings

Fig. 1 is a flowchart illustrating an example of a process of a critical process parameter analysis method in accordance with a first embodiment of the present invention.
Fig. 2 is a view illustrating an example of a manufacturing process for manufacturing a cell preparation in accordance with a second embodiment of the present invention.
Fig. 3 is a view illustrating evaluation of control difficulties of respective motions and specification of an operation in accordance with the second embodiment of the present invention.
Fig. 4 is a view illustrating evaluation of control difficulties of the respective motions and evaluation of a response variability in accordance with the second embodiment.
Fig. 5 shows a table in which operation difficulties of respective partial processes are arranged in accordance with the second embodiment of the present invention.
Fig. 6 shows a table in which response variabilities of the respective partial processes are arranged in accordance with the second embodiment of the present invention.
Fig. 7 shows a table in which variability indexes of the respective partial processes are arranged in accordance with the second embodiment of the present invention.
Fig. 8 is a block diagram illustrating a configuration of a main part of a critical process parameter analysis device in accordance with a third embodiment of the present invention.
Fig. 9 is a conceptual diagram illustrating an example of a case where the critical process parameter analysis device is applied.

### Description of Embodiments

### First Embodiment

The following will describe details of an embodiment of the present invention. The description in the present embodiment will discuss a critical process parameter analysis method for specifying a critical process parameter candidate which affects an important quality characteristic of a product in a manufacturing process of a product.

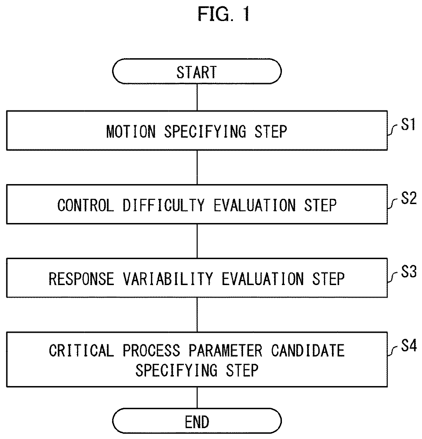

Fig. 1 is a flowchart illustrating an example of a process of a critical process parameter analysis method in accordance with the present embodiment. As shown in Fig. 1, an extraction method in accordance with the present embodiment includes a motion specifying step S1, a control difficulty evaluation step S2 (variation evaluation step), a response variability evaluation step S3 (variation evaluation step), and a critical process parameter candidate specifying step S4.

The motion specifying step S1 is a step of specifying, for each of processes in the manufacturing process, a plurality of motions included in the process. Specifically, the motion specifying step S1 may be configured such that, for example, in a case where motions carried out in each of the processes are motions that a device is caused to carry out in order to execute the process, the motions carried out by the device may be specified as the motions in the process. Alternatively, the motion specifying step S1 may be configured such that, for example, in a case where motions carried out in each of the processes are motions which are to be carried out by a human, the motions carried out by the human may be specified as the motions in the process. Preferably, when specifying the motions, the motion specifying step S1 subdivides the motions specifically as much as possible (e.g., "opening the door", "holding the container").

The control difficulty evaluation step S2 is a step of evaluating a degree of variation in a system input which is input into a subject system from an outside of the subject system in accordance with a corresponding one of motion parameters which are set to execute the respective plurality of motions included in the manufacturing process of the product, the subject system being a subject of the plurality of motions and including a response field exhibiting a response to each of the motions. The "response field" herein refers to a field indicative of a response to each of the motions included in the manufacturing process of the product. For example, in a case where the subject is manufacturing of a cell preparation, the response field is a field where reaction of cells takes place, and may include cells, a liquid, and/or the like. For another example, in a case where the subject is manufacturing of a chemical product, including a polymerization reaction process, the response field is a field where reaction of a chemical substance takes place, and may include a solid, a liquid, and/or the like. The "subject system" herein refers to a system which is the subject of the plurality of motions included in the manufacturing process of the product and which includes the above-described response field. For example, in a case where the subject is manufacturing of a cell preparation, the subject system may be a culture layer, a tank, a test tube, or the like in each of which cells and a liquid are contained. The variation in the system input may be variation in a motion output which is actually output with respect to the subject system.

The control difficulty evaluation step S2 may evaluate control difficulties of the respective motions included in each of the processes. In this case, for each of the motions specified by the motion specifying step S1, a control difficulty which is based on a degree of a difference between (a) a control target of a corresponding one of the motion parameters which are set for the respective motions and (b) a control state of an actual motion parameter of the each of the motions.

Specifically, first, control target values are set for the respective motions. Each of the control target values is set for a motion parameter which can affect an important index reflecting an important quality characteristic of the product. The motion parameter is a parameter of a motion affecting an environment of the response field which is the subject of the motions in each of the processes. Examples of the motion parameter include a pH, an oxygen concentration, a temperature, and the like. Furthermore, the motion parameter may include duration time of the motion. Hereinafter, the duration time of the motion may also be referred to as "motion time". Moreover, the motion parameter may include a mechanical parameter for the motion. For example, in a case where the motion is the motion of "holding container", the mechanical parameter for the motion may be a speed of holding the container. The control target of the motion parameter is set for each of the motions.

Next, for each of the motions, calculation is carried out to yield an index indicative of a degree of a difference (hereinafter, referred to as a "first difference") between (a) the control target value having been set and (b) a control state in actual execution of each of the plurality of motions. Specifically, first, for each of the motion parameters, a range of a controllable value in execution of the motion is determined. Next, for each of the motion parameters, calculation is carried out to yield an index indicative of a scale difference of a difference between (a) the control target value having been set and (b) a range of the controllable value in execution of the motion (hereinafter, such a range will be referred to as a "controllable range"). Here, the controllable range is a range of a value which can be actually output when the motion is executed with the control target value having been set. Hereinafter, the index indicative of the scale difference of the difference between (a) the control target value for each of the motions and (b) the controllable range will be referred to as a "control difficulty index". The control difficulty index is an index which is calculated on the basis of a degree of the first difference.

The control difficulty index may be calculated in the following manner, for example. First, among the values included in the controllable range, a value which is most greatly different from the control target value is specified, and then a value (hereinafter, referred to as a "maximum difference value") obtained by subtracting the control target value from such a value is calculated. Next, indexes which are the control target value and the maximum difference value expressed by a power are calculated. Then, the index of the control target value is subtracted from the index of the maximum difference value, so that a control difficulty index is calculated. As one example, in a case where, for a motion parameter for the "speed of holding the container" in the motion of "holding container", the control target is set at 0.1 m/s and the controllable range is 0.09 m/s to 0.12 m/s, the maximum difference value is as follows: 0.12 - 0.1 = 0.02 m/s. For example, when expressed by a power having 10 as a base, the control target value and the maximum difference value are respectively 1×10⁻¹ m/s and 2×10⁻² m/s. Thus, the control difficulty index is calculated as "-1", which is a value obtained by subtracting (a) "-1" which is an index of the control target value from (b) "-2" which is an index of the maximum difference value. In the above explanation, the control target value and the maximum difference value are expressed by a power having 10 as a base. Alternatively, a control difficulty index may be calculated by expressing these values by a power having a numerical value other than 10 as a base.

In another example, the control difficulty index may be calculated by dividing the above-described maximum difference value by the control target value. In one example, in a case of the "speed of holding a container" of the motion of "holding container", 0.2, which is obtained by dividing (a) "0.02 m/s" that is the maximum difference value by (b) "0.1 m/s" that is the control target value, may be obtained as the control difficulty index.

Then, by using the control difficulty indexes thus calculated, the control difficulty evaluation step S2 evaluates control difficulties of the respective motion parameters. Specifically, a motion parameter having a maximum control difficulty index may be specified, and the control difficulty index of that motion parameter may be specified as the control difficulty index indicative of the control difficulty of that motion. Alternatively, the motion parameters may be ordered in a decreasing order of the control difficulty indexes.

As discussed above, the control difficulty evaluation step S2 evaluates, for each of the motion parameters, the control difficulty based on the degree of the first difference between (a) the control target value set in execution of a corresponding one of the plurality of motions and (b) the actual control state. Then, by using the control difficulties regarding the plurality of motions, a control difficulty of the process including the plurality of motions is evaluated. The evaluation of the control difficulty is carried out for each process.

The above-described example has dealt with the case where a single value is set as the control target value. In an aspect of the present invention, in a case where a numerical range is set as the control target value, the control difficulty index may be calculated as an index indicative of a difference between (a) a scale of a range of the control target value and (b) a scale of a controllable range in execution of the motion.

The response variability evaluation step S3 is a step of evaluating a degree of variation in a system output which is output from the subject system in response to each of the motions. Specifically, the response variability evaluation step S3 evaluates, for each of the plurality of motions, a response variability indicative of a degree of variation in an output from the subject system. In this case, the response variability based on the variation in the system output in response to each of the motions specified by the motion specifying step S1 is evaluated for each of the plurality of motions. Specifically, calculation is carried out to yield an index indicative of a scale of a difference (hereinafter, which is also referred to as a "second difference") between a motion (input) with respect to the subject system (response field) and an response output from the subject system (response field). Hereinafter, the index indicative of the scale of the second difference will be referred to as a "response variability index". For example, in a case where the present invention is applied to a manufacturing process for manufacturing a cell preparation, a period of time of a cell response can be used as an index for calculating the response variability index.

The response variability index may be calculated in the following manner. That is, a motion input (motion input value) with respect to the subject system and a response output value from the subject system are calculated as indexes expressed by a power, and then a response variability index is obtained by subtracting (a) the index of the response output value from the subject system from (b) the index of the motion input. For example, consider a case where the present invention is applied to the manufacturing process for manufacturing the cell preparation. Then, when "death (apoptosis/necrosis)" is set as a cell response output in response to a motion (input) with respect to the subject system, it is assumed that a period of time (hereinafter, referred to as "motion time") necessary to carry out a motion is 10 seconds and a period of time (hereinafter, referred to as "cell response time") taken until cells exhibit a death phenotype in response to a motion input (i.e., occurrence of apoptosis) is an hour (i.e., 3600 seconds). In this case, the motion time and the cell response time are respectively 1×10¹ seconds and 3.6×10³ seconds when expressed by a power having 10 as a base. Thus, the response variability index is calculated as "-2", which is obtained by subtracting (a) "3" that is an index of the cell response time from (b) "1" that is an index of the motion time. In the above explanation, the motion time and the cell response time are expressed by a power having 10 as a base. Alternatively, a response variability index may be calculated by expressing these values by a power having a numerical value other than 10 as a base.

The response variability evaluation step S3 then evaluates the response variability for each process by using the response variability indexes thus calculated. Specifically, among the motions included in each process, a motion having a maximum response variability index may be specified, and the response variability index of that motion may be specified as a response variability index indicative of a response variability of the process. Alternatively, the motions may be ordered in a descending order of the response variability indexes.

As discussed above, the response variability evaluation step S3 evaluates, for each of the plurality of motions, the response variability based on the second difference of the each of the plurality of motions. Then, by using the response variabilities related to the plurality of motions, the response variability of the process including the plurality of motions is evaluated. The evaluation of the response variability is carried out for each process.

The critical process parameter candidate specifying step S4 is a step of specifying, on the basis of the control difficulties and the response variabilities, a critical process parameter candidate from among motion parameters included in the motions in the manufacturing process for the product. Hereinafter, each of the plurality of processes included in manufacturing of the product will be called a "partial process". By using (a) the control difficulty index of each of the partial processes calculated by the control difficulty evaluation step S2 and (b) the response variability index of each of the partial processes calculated by the response variability evaluation step S3, the critical process parameter candidate specifying step S4 calculates, for each of the partial processes, a variability index indicating an effect given to the important index. The variability index thus calculated is an index indicating a variability of the manufacturing system of the product. There is no particular limitation on a method for calculating the variability index. For example, the variability index may be calculated by obtaining a linear sum of the control difficulty index and the response variability index while assigning a given weight. In a specific example, for each of the motion parameters, a numerical value calculated by obtaining a linear sum of the control difficulty index calculated by the control difficulty evaluation step S2 and the response variability index calculated by the response variability evaluation step S3 while assigning a weight of 1:1. The critical process parameter candidate specifying step S4 specifies, as a critical process parameter candidate, a motion parameter whose calculated variability index is equal to or more than a given value.

As discussed above, the critical process parameter analysis method in accordance with the present embodiment calculates a variability index of a system on the basis of (a) a control difficulty index indicative of stability instability of an input value of each motion and (b) a response variability index indicative of instability of an output from the subject system (response field) in response to an input of the motion. Then, on the basis of the variability index thus calculated, a motion parameter which greatly affects the important index is automatically specified as the critical process parameter candidate. With the above configuration, it is possible to automatically specify the critical process parameter candidate while reflecting (a) stability/instability of the input value of each motion and (b) instability of the output from the subject system (response field) in response to the input of the motion.

Note that, in a case where an aspect of the critical process parameter analysis method of the present invention is applied to a manufacturing process including partial processes each having a predetermined motion(s), the motion specifying step S1 can be omitted.

In the configuration of the present embodiment, the control difficulties and the response variabilities are calculated for the respective motions. However, the critical process parameter analysis method in accordance with the present invention is not limited to this. For example, a series of motions for which the subject system exhibits the same response may be defined as a motion group, and a control difficulty and a response variability may be calculated for each motion group. This makes it possible to reduce the number of subjects for specifying the critical process parameter candidate.

### Second Embodiment

The following description will discuss another embodiment of the present invention. Note that, for convenience, members having identical functions to those of the foregoing embodiment are given identical reference signs, and their descriptions will be omitted. The present embodiment will discuss, as a specific example of the critical process parameter analysis method explained in the first embodiment, a critical process parameter analysis method in a manufacturing process for manufacturing a cell preparation.

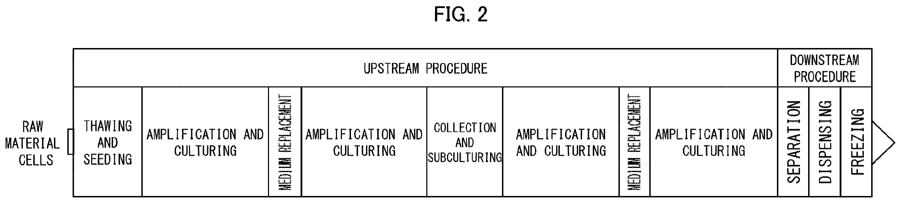

Fig. 2 is a view illustrating an example of a manufacturing process for manufacturing a cell preparation. The present embodiment will explain a critical process parameter analysis method in the manufacturing process of the cell preparation shown in Fig. 2, the manufacturing process including partial processes, specifically, a thawing and seeding process, an amplification and culturing process (cell culturing process), a medium replacement process, a collection and subculturing process, a separation process, a dispensing process, and a freezing process. This critical process parameter analysis method may include other process(es).

### Motion Specifying Step

In the critical process parameter analysis method in accordance with the present embodiment, first, a plurality of motions included in each partial process are specified for the each partial process (motion specifying step). For example, for the collection and subculturing process, the motions such as "transferring to incubator", "holding outer door of incubator", and "opening outer door of incubator" are specified.

### Control Difficulty Evaluation Step for Each Motion

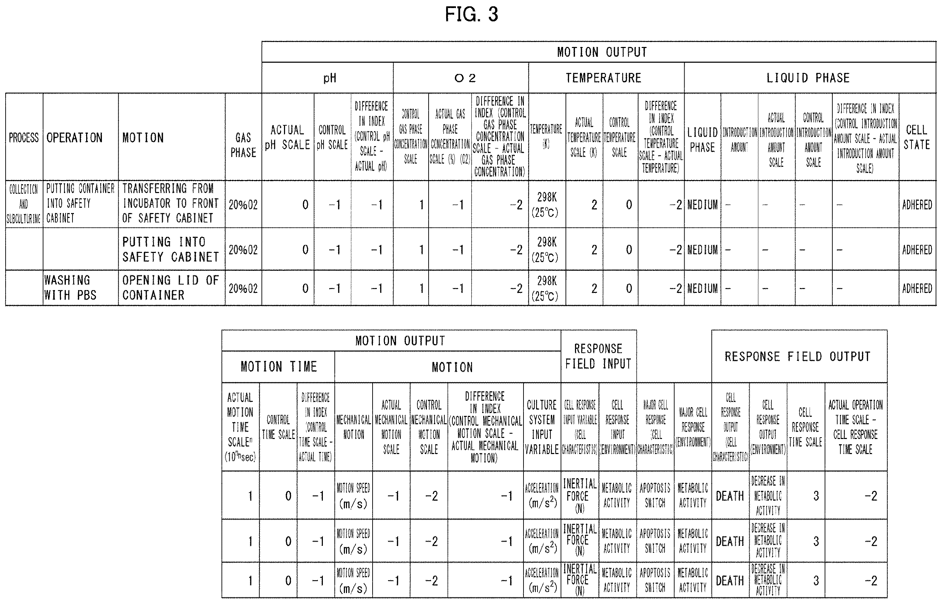

Next, a control difficulty for each of the motions included in each partial process is evaluated. In the description below, the collection and subculturing process will be discussed as an example. Fig. 3 is a view illustrating evaluation of control difficulties of the respective motions and specification of an operation (described later) in the collection and subculturing process. Illustrated in Fig. 3 is a part of the motions in the collection and subculturing process.

This step first sets a motion parameter (process parameter) for each of the motions specified by the motion specifying step. The motion parameter is a parameter which serves as an input with respect to a response field (i.e., cells) and which can affect an important index (e.g., a viability of the cells) reflecting an important quality characteristic of the cell preparation to be manufactured. In an example shown in Fig. 3, "pH", "O2 (oxygen concentration)", "temperature", "liquid phase", "motion time", and "motion" are set as motion parameters.

Next, a control target value is set for each motion parameter for the respective motion. In the example shown in Fig. 3, the control target values are set for the respective motion parameters of "pH", "O2 (oxygen concentration)", "temperature", "liquid phase", "motion time", and "motion".

Next, calculation is carried out to yield numerical values of indexes expressing, by a power having 10 as a base, the control target values having been set. In the present embodiment, a numerical value of an index expressed by a power having 10 as a base will be called a "scale". For example, assume that the control target value for the motion parameter "pH" for the motion (hereinafter, referred to as "motion 1") of "transferring from incubator to front of safety cabinet" is set at "7". In this case, "7" expressed by a power having 10 as a base is "7×10⁰". Thus, a scale of the control target value for the motion parameter "pH" in motion 1 is calculated as "0". In this case, a scale of the calculated control target value corresponds to "0" in the column "actual pH scale" in the table shown in Fig. 3.

Next, calculation is carried out to yield a numerical value of an index expressing, by a power having 10 as a base, a maximum difference value between (a) a value included in a range (i.e., a controllable range) of a controllable value of each motion parameter in execution of the motion and (b) the control target value. In other words, calculation is carried out to yield a scale of the maximum difference value. For example, in a case where, for the motion parameter "pH" of motion 1, a range "6.9 to 7.2" is set as a controllable pH range, the maximum difference value is "0.2 (= 7.2 - 7)", and is "2×10-¹" when expressed by a power having "0.2" as a base. Thus, a scale of the maximum difference value is calculated as "-1". The scale of the maximum difference value thus calculated corresponds to "-1" in the column "control pH scale" in the table shown in Fig. 3.

Next, calculation is carried out to yield a value by subtracting the scale of the control target value from the scale of the maximum difference value. For example, for the motion parameter "pH" of motion 1, the scale of the maximum difference value is "-1" and the scale of the control target value is "0". Thus, calculation of "-1 (= -1 - 0)" is carried out. The calculated value corresponds to "-1" in the column "difference in index (control pH scale - actual pH)" in the table shown in Fig. 3. The value obtained by subtracting the scale of the control target value from the scale of the maximum difference value is an index indicative of a difference between (a) the scale of the control target value of the motion parameter and (b) the scale of the controllable range of the motion parameter in execution of the motion, that is, a control difficulty index of the motion parameter.

### Operation Specifying Step

Next, in each partial process, a series of motions for which the subject system exhibits the same response is specified as a single motion group. In the following description, the above-described motion group will be referred to as an "operation". In this step, first, for each of the motions, an input with respect to the response field (hereinafter, such an input will be referred to as a "response field input") is specified. For example, in the case of motion 1, an inertial force is input into the response field by a mechanical motion, and a metabolic activity changes due to a change in the environment (i.e., a pH, an oxygen concentration, a temperature, and/or the like). Therefore, as response field inputs, an "inertial force" and a "metabolic activity" are specified. In this case, the response field inputs thus specified correspond to (a) the "inertial force (N)" in the column "cell response input variable (cell characteristic)" and (b) the "metabolic activity" in the column "cell response input (environment)" in the table shown in Fig. 3.

Next, responses of the response field in response to the specified response field inputs are specified. For example, in the case of motion 1, a response exhibited by the cells in response to the "inertial force" is specified as an "apoptosis switch", and a response exhibited by the cells in response to the "environment" is specified as a "metabolic activity". In this case, the specified responses correspond to the "apoptosis switch" in the column "major cell response (cell characteristic)" and the "metabolic activity" in the column "major cell response (environment)" in the table shown in Table 3.

Next, for each of the motions, a response output of the response field is specified. For example, in the case of motion 1, the cells die as a result of activation of the apoptosis switch due to the inertial force. Therefore, "death" is specified as a response field output. Furthermore, since the metabolic activity of the cells decreases due to the change in the environment, "decrease in metabolic activity" is specified as a response output. In this case, the specified response field outputs correspond to (a) the "death" in the column "cell response output (cell characteristic)" and (b) the "decrease in metabolic activity" in the column "cell response output (environment)" in the table shown in Fig. 3.

Next, for each of the motions, calculation is carried out to yield a numerical value of an index expressing, by a power having 10 as a base, a period of time (hereinafter, referred to as "response time") taken until the response field (cells) outputs a response after inputting of a motion. In other words, calculation is carried out to yield a scale of the response time. In a case where a plurality of responses are given as a response of the response field (cells), a period of response time of, among the plurality of responses, a response having a shorter response time is used to calculate a scale of the response time. For example, with regard to motion 1, assume that a period of time taken until a response of, among the death and the decrease in metabolic activity, the one which gives a response in a shorter period of time is 3600 seconds. In this case, "3600" expressed by a power having 10 is "3.6×10³". Thus, a scale of the response time is calculated as "3". In this case, the scale of the response time having been calculated corresponds to "3" in the column "cell response time scale" in the table shown in Fig. 3.

Next, a series of motions for which the subject system exhibits the same response is arranged as a single motion group (i.e., an operation). Specifically, a series of motions having the same cell response output and the same cell response time scale is specified as a single operation. For example, in the example shown in Fig. 3, the motion of "transferring from incubator to front of safety cabinet" (i.e., motion 1) and the motion of "putting into safety cabinet" (hereinafter, this motion will be referred to as "motion 2") are arranged as the same operation, since these motions respectively involve "death" and "decrease in metabolic activity" as the cell response outputs from the subject system and both have "3" as the cell response time scales. Furthermore, in the present embodiment, motions having different degrees of cleanliness in the environment in which the motions are executed are arranged as different motions. For example, motions 1 and 2 are motions carried out outside the safety cabinet, and the motion of "opening lid of container" shown in Fig. 3 (hereinafter, this motion will be referred to as "motion 3") is a motion carried out inside the safety cabinet. Therefore, motion 3 is different from motions 1 and 2 in the degree of cleanliness in the environment in which the motions are executed. Therefore, although motion 3 is identical to motions 1 and 2 in the cell response output from the subject system and the cell response time scale, motion 3 is not arranged as the same operation as motions 1 and 2.

### Control Difficulty Evaluation Step for Each Operation

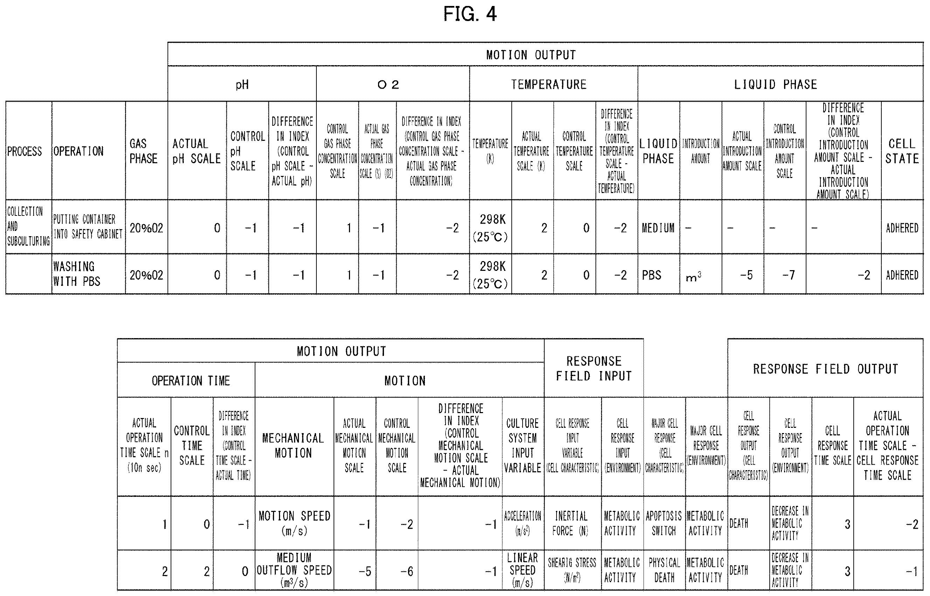

Next, control difficulties of the respective motion parameters are evaluated for each operation. Fig. 4 is a view illustrating evaluation of control difficulties of the respective motions and evaluation of a response variability (described later) in the collection and subculturing process. Illustrated in Fig. 4 is a part of the operations in the collection and subculturing process.

This step first sets, for each of the operations, control target values of the respective motion parameters as shown in Fig. 4. Each of the motion parameters is the motion parameter having been set in evaluating the control difficulty of the respective motion. In this process, in a case where a plurality of motions included in a single operation have different control target values, a control target value of a motion whose motion parameter has a largest variation is set as a control target value of that operation. Next, calculation is carried out to yield a numerical value of an index expressing, by a power having 10 as a base, the control target value having been set. That is, calculation is carried out to yield a scale of the respective control target value having been set is calculated. For example, a scale of a control target value calculated for "pH" corresponds to the numerical value in the column "actual pH scale" in the table shown in Fig. 4.

Next, for each of the motion parameters, calculation is carried out to yield an index indicative of a difference between (a) the scale of the control target value of the motion parameter and (b) a scale of a range (i.e., a controllable range) of a controllable value in execution of the operation, i.e., a control difficulty index. Specifically, first, calculation is carried out to yield a numerical value of an index expressing, by a power having 10 as a base, a maximum difference value between the control target value of the motion parameter and the controllable range, that is, a scale of the maximum difference value. For example, a scale of a maximum difference value calculated for "pH" corresponds to the numerical value in the column "control pH scale" in the table shown in Fig. 4.

Next, calculation is carried out to yield, as the control difficulty index, a value obtained by subtracting the scale of the control target value from the scale of the maximum difference value. For example, the value calculated for "pH" corresponds to the numerical value in the column "difference in index (control pH scale - actual pH)" in the table shown in Fig. 4.

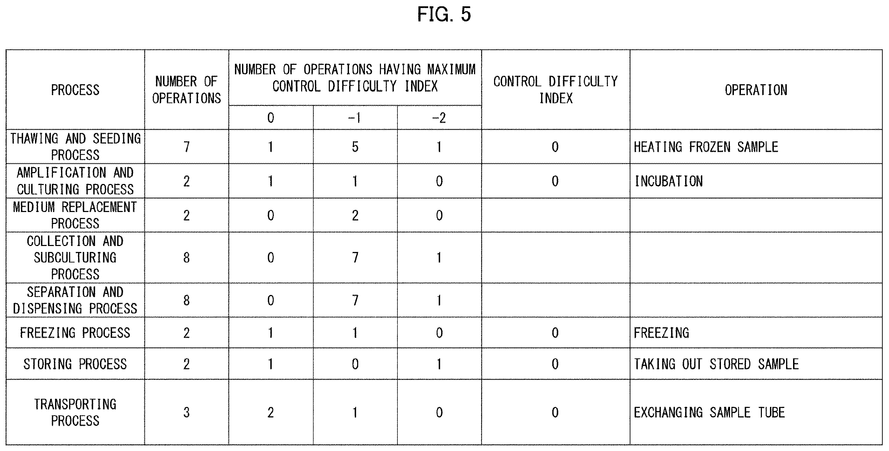

Next, control difficulties of the respective operations in each partial process are evaluated. This will be explained with reference to Fig. 5. Fig. 5 shows a table in which operation difficulties of the respective partial processes are arranged by using the control difficulty indexes calculated in the above-described manner. As shown in Fig. 5, for example, for the "thawing and seeding process", seven operations are arranged. Furthermore, it is evaluated such that (a) among the operations, the number of operations whose maximum control difficulty index of a motion parameter is "0" is one, the number of operations whose maximum control difficulty index of a motion parameter is "-1" is five, and the number of operations whose maximum control difficulty index of a motion parameter is "-2" is one and (b) an operation having a highest control difficulty is "heating frozen sample".

### Response Variability Evaluation Step

Next, response variability indexes of the respective operations are evaluated. Specifically, first, actual operation time scales of the respective operations are specified. An actual motion time scale of each operation is calculated as a numerical value of an index expressing, by a power having 10 as a base, a total of periods of motion time of a series of motions included in each operation. The calculated value corresponds to the numerical value in the column "actual motion time scale" in the table shown in Fig. 4.

Next, as a response variability index of the operation, a value obtained by subtracting a cell response time scale of the operation from the calculated actual motion time scale is calculated. The calculated response variability index corresponds to the numerical value in the column "actual operation time scale - cell response time scale" in the table shown in Fig. 4.

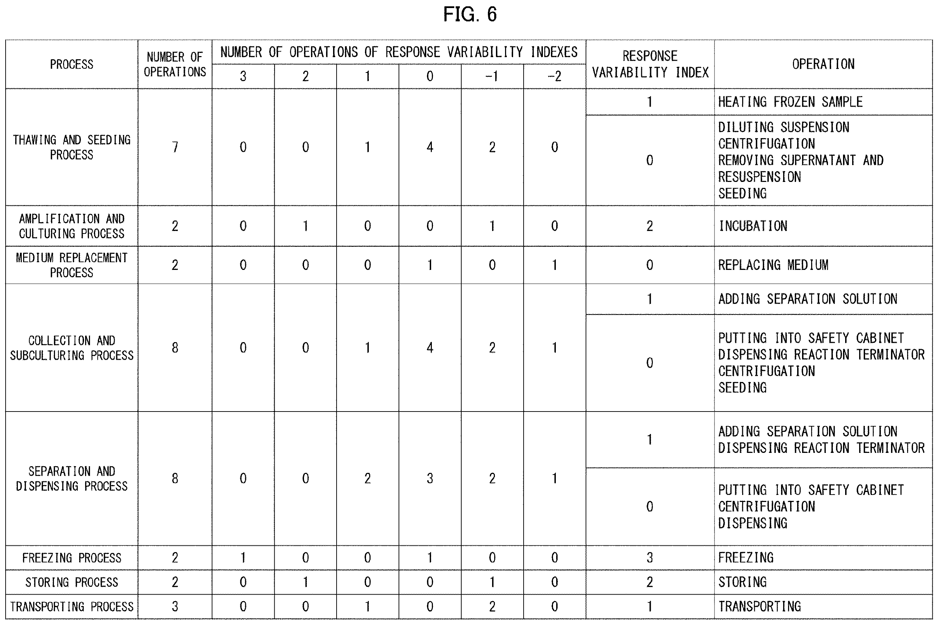

Next, response variabilities of the respective operations are evaluated. This will be explained with reference to Fig. 6. Fig. 6 shows a table in which response variabilities of the respective partial processes are arranged by using the response variability indexes calculated in the above-described manner. For example, as shown in Fig. 6, for the "thawing and seeding process", it is evaluated such that (a) among the operations, the number of operations whose maximum response variability index is "1" is one, the number of operations whose maximum response variability index is "-0" is four, and the number of operations whose maximum response variability index is "-1" is two and (b) an operation having a highest response variability is "heating frozen sample".

### Critical Process Parameter Candidate Specifying Step

Next, a critical process parameter candidate in a manufacturing process for manufacturing a cell preparation is specified. The following will explain two patterns, i.e., (a) a method for specifying the critical process parameter candidate by calculating variability indexes of the respective operations and (b) a method for specifying the critical process parameter candidate by calculating variability indexes of the respective motions.

The method for specifying the critical process parameter candidate by calculating the variability indexes of the respective operations is carried out as follows. First, for each of the partial processes, calculation is carried out for each of the operations to yield a numerical value by obtaining a linear sum of (a) the control difficulty index calculated for each of the motion parameters by the control difficulty evaluation step for each of the operations and (b) the response variability index calculated by the response variability evaluation step, while assigning a given weight. The description in the present embodiment will deal with an example in which calculation is carried out to yield, as a variability index, a numerical value obtained by a linear sum of the control difficulty index and the response variability index while assigning a weight of 1:1. For example, for the operation of "putting container into safety cabinet" in the "collection and subculturing process", a highest control difficulty index among those of the motion parameters is "-1" for "pH", "operation time", and "motion" and the response variability index is "-2". Thus, the variability index is calculated as follows: 1 × (-1) + 1 × (-2) = -3.

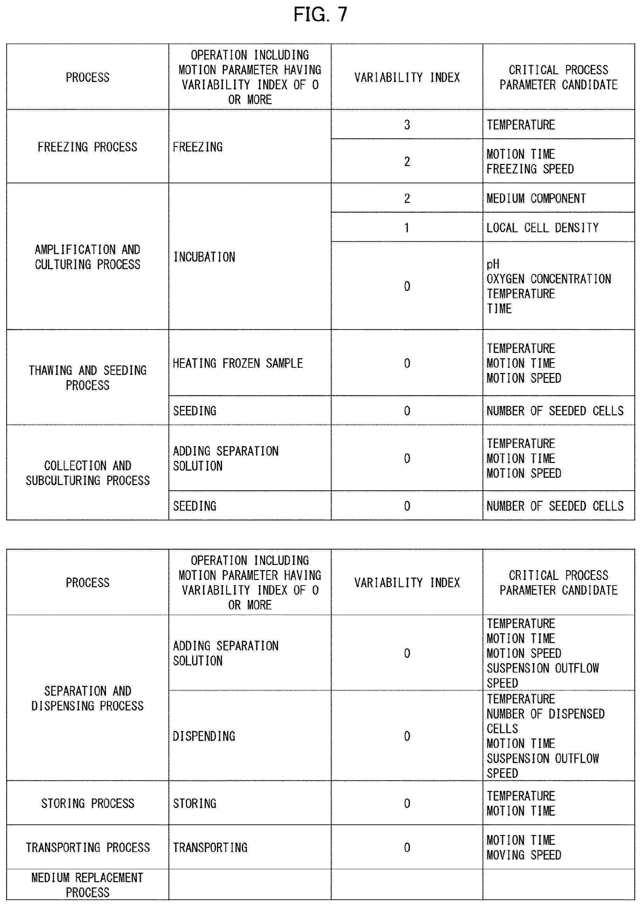

Next, a motion parameter whose variability index having been calculated is equal to or more than a given value is specified as a critical process parameter candidate. Fig. 7 shows a table indicating variability indexes of the respective partial processes. In the present embodiment, a motion parameter whose variability index is equal to or more than 0 is specified as the critical process parameter candidate. The example shown in Fig. 7 shows that, for example, for the "collection and subculturing process", "temperature", "motion time", and "motion speed" in the operation of "adding separation solution" as well as "number of seeded cells" in the operation of "seeding" are specified as critical process parameter candidates.

The method for specifying the critical process parameter candidate by calculating variability indexes of the respective motions is carried out as follows. For each of the motion parameters, calculation is carried out to yield, as a variability index, a numerical value obtained by a linear sum of (a) the control difficulty index calculated for each of the partial processes by the control difficulty evaluation step carried out for each of the motions and (b) the response variability index calculated by the response variability evaluation step, while assigning a given weight. Then, a motion parameter whose variability index having been calculated is equal to or more than a given value is specified as a critical process parameter candidate.

In a case where the critical process parameter candidate is specified by the method for specifying the critical process parameter candidate by calculating the variability indexes of the respective operations, calculation for yielding control difficulty indexes of the respective operations may be carried out and thus calculation for yielding control difficulty indexes of the respective motions is not necessary.

Further, the present embodiment also encompasses a training method of production manufacturing designing using an instruction manual and/or the like including the above-discussed critical process parameter analysis method.

### Third Embodiment

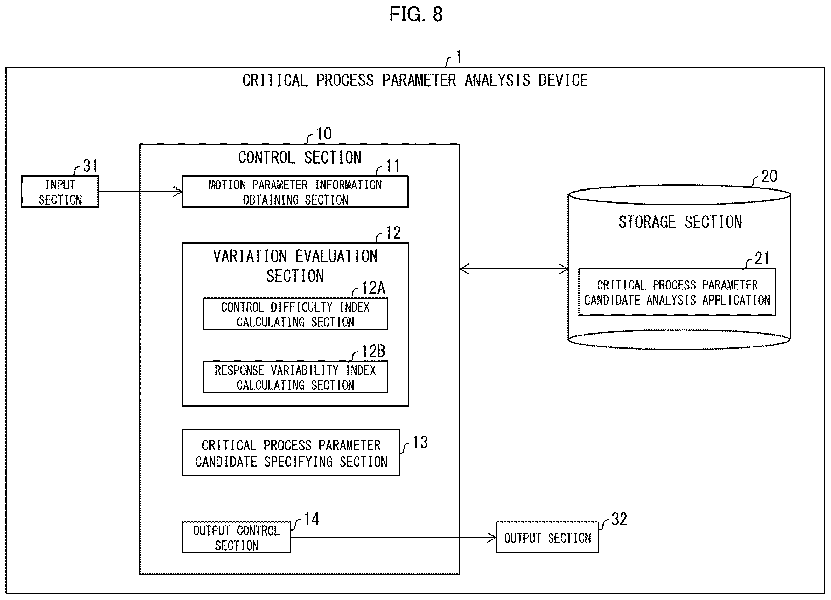

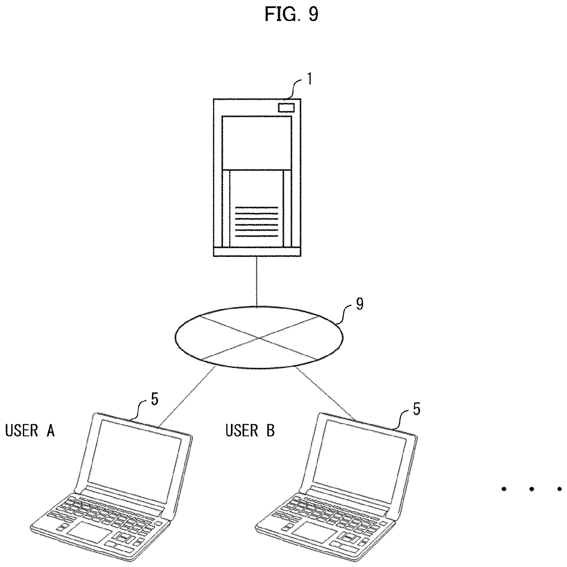

The following description will discuss another embodiment of the present invention. The critical process parameter analysis method discussed in the first and second embodiments may be executed by one or more computers. The description in the present embodiment will discuss a critical process parameter analysis device which executes the critical process parameter analysis method explained in the first embodiment. Fig. 8 is a block diagram illustrating a configuration of a main part of a critical process parameter analysis device 1 in accordance with the present embodiment. Fig. 9 is a conceptual diagram illustrating an example of a case where the critical process parameter analysis device 1 is applied.

The critical process parameter analysis device 1 is a device which specifies a critical process parameter candidate from among motion parameters which are to be set to carry out motions included in a manufacturing process of a product. As shown in Fig. 8, the critical process parameter analysis device 1 includes: a control section 10; a storage section 20 in which various kinds of data used by the critical process parameter analysis device 1 is stored; an input section 31 which accepts input operation carried out with respect to the critical process parameter analysis device 1; and an output section 32 which displays various kinds of information.

The control section 10 comprehensively controls the sections in the critical process parameter analysis device 1. The control section 10 includes a motion parameter information obtaining section 11, a variation evaluation section 12, a critical process parameter candidate specifying section 13, and an output control section 14.

The motion parameter information obtaining section 11 obtains information about the motion parameters. The information about the motion parameters includes: the number of partial processes included in the manufacturing process of the product; motions included in each of the partial processes; the motion parameters of the respective motions; control target values set for the motion parameters for the respective motions; controllable ranges of the respective motion parameters; periods of motion time of the respective motions; periods of response time of the response field in response to the respective motions; and/or the like. The motion parameter information obtaining section 11 may obtain the information about the motion parameters by receiving the information about the motion parameters input thereto via the input section 31. Further, as shown in Fig. 9, the motion parameter information obtaining section 11 may obtain the information about the motion parameters from an information processing terminal 5 of a user over a network 9.

The motion parameter information obtaining section 11 may obtain, as at least a part of the information about the motion parameters, information obtained by prediction carried out by a prediction means such as computer simulation.
In this case, the later-described variation evaluation section 12 may evaluate, on the basis of information including virtual data obtained by using the prediction means, (a) a degree of variation in a system input which is input to a subject system including the response field from the subject system and (b) a degree of variation in a system output which is output from the subject system in response to a motion. The prediction involving use of the prediction means may be carried out by the motion parameter information obtaining section 11 itself.

The variation evaluation section 12 evaluates, for each of the motions, (a) the degree of the variation in the system input which is input to the subject system including the response field from the subject system and (b) the degree of the variation in the system output which is output from the subject system in response to the motion. The variation evaluation section 12 includes a control difficulty index calculating section 12A and a response variability index calculating section 12B.

The control difficulty index calculating section 12A calculates a control difficulty index indicative of a degree of variation in the system input. Specifically, by using the information obtained by the motion parameter information obtaining section 11, the control difficulty index calculating section 12A calculates, for each of motion parameters of motions included in a partial process, a control difficulty index indicative of a scale difference of a difference between (a) a control target value set by the user and (b) a controllable range in execution of the motion. As a method for calculating the control difficulty index, the method explained in the first embodiment is used. The control difficulty index calculating section 12A outputs the calculated control difficulty index to the critical process parameter candidate specifying section 13.

The response variability index calculating section 12B calculates a response variability index indicative of a degree of variation in the system output which is output from the subject system in response to a motion. Specifically, by using the information obtained by the motion parameter information obtaining section 11, the response variability index calculating section 12B calculates, for each of the motions included in the partial process, a response variability index indicative of a scale of a difference between (a) a motion (input) with respect to the subject system and (b) a response output from the subject system (response field). As a method for calculating the response variability index, the method explained in the first embodiment is used. The response variability index calculating section 12B outputs the calculated response variability index to the critical process parameter candidate specifying section 13.

By using an evaluation result obtained by evaluating, for each of the motions, a degree of variation in the system input and a degree of variation in the system output (i.e., the control difficulty index which is output from the control difficulty index calculating section 12A and the response variability index which is output from the response variability index calculating section 12B), the critical process parameter candidate specifying section 13 specifies a critical process parameter candidate from among the motion parameters included in the manufacturing process of the product. Specifically, for each of the motions, the critical process parameter candidate specifying section 13 calculates a variability index by obtaining a linear sum of (a) a maximum control difficulty index among the motion parameter control difficulty indexes and (b) a response variability index of the motion while assigning a given weight. Then, the critical process parameter candidate specifying section 13 specifies, on the basis of the calculated variability index, a critical process parameter candidate from among the motion parameters included in the manufacturing process of the product. For example, the critical process parameter candidate specifying section 13 specifies, as the critical process parameter candidate, a motion parameter whose variability index is equal to or more than a given value.

The output control section 14 controls the output section 32 so as to cause the output section 32 to output the critical process parameter candidate specified by the critical process parameter candidate specifying section 13.

The variation evaluation section 12 and the critical process parameter candidate specifying section 13 may carry out the motion by using a critical process parameter candidate analysis application 21 including the critical process parameter analysis program which is stored in the storage section 20 in advance.

The critical process parameter analysis device 1 in accordance with an aspect of the present invention may provide information including a motion parameter which can be the specified critical process parameter candidate to an external terminal device over a communication network (e.g., the Internet).

### Fourth Embodiment

The following will describe another embodiment of the present invention. The critical process parameter analysis method explained in the first and second embodiments can also be used to change and/or improve the critical process parameter. In an example explained in the present embodiment, the critical process parameter analysis method explained in the first and second embodiments is applied to change and/or improve the critical process parameter candidate.

In the example shown in Fig. 7, "temperature" in the operation of "adding separation solution" in the "collection and subculturing process" is specified as a critical process parameter candidate. The reason for this is as follows. That is, since a separation solution (hereinafter, referred to as "separation solution A") used in the "collection and subculturing process" is such a separation solution that exerts a desired effect when transferred into an environment at a temperature of 37°C after being added at room temperature, it is necessary to change the temperature during use of the separation solution.

Assume that there is separation solution B, which is a separation solution that can be used under a certain environment, specifically, a separation solution that exerts a desired effect at room temperature after being added at room temperature. In this case, in the operation of "adding separation solution", the separation solution to be used can be changed from separation solution A to separation solution B. In this case, critical process parameter candidates selected when the separation solution is changed from separation solution A to separation solution B are specified by using the critical process parameter analysis method explained in the first and second embodiments. When separation solution B is used, it is not necessary to change the temperature, unlike when separation solution A is used. Therefore, the control difficulty index for "temperature" in the operation of "adding separation solution" becomes smaller, and consequently the variability index becomes smaller. Thus, it is possible to indicate that, for the "collection and subculturing process", "temperature" in the operation of "adding separation solution" is removed from a list of the critical process parameter candidates. Meanwhile, if the use of separation solution B arises the need for a procedure and/or a motion unnecessary when separation solution A is used, it is possible to indicate whether or not a parameter associated with the procedure and/or motion is specified as a critical process parameter candidate. Consequently, a designer of the manufacturing process can check a change in the critical process parameter candidate which change is caused by changing of the separation solution from separation solution A to separation solution B. Further, in consideration of a result of the checking, the designer can change and/or improve the critical process parameter candidate.

The above description has dealt with, as an example, a situation in which changing of the separation solution is considered in the "collection and subculturing process". However, the present invention is not limited to this situation. Alternatively, for example, it is possible to indicate that "motion speed" is removed from the list of the critical process parameter candidates by mechanizing motions which have been manually conducted and making a motion speed therefor constant. As discussed above, application of the critical process parameter analysis method makes it possible to check how a change in each process affects the critical process parameter candidates before the change is actually applied.

As discussed above, in a case where at least a part of the elements in the manufacturing process is changed, the critical process parameter analysis method explained in the first and second embodiments may be applied to (a) a case in which the element(s) has/have not been changed yet and (b) a case in which the element(s) has/have been already changed. Then, it is possible to check a change in the critical process parameter which change is caused by changing of the manufacturing process and to consider whether to apply the change. Consequently, the designer of the manufacturing process can check how the critical process parameter candidate is changed by the change in the manufacturing process before the change in the manufacturing process is actually applied. Thus, in consideration of a result of the checking, the designer of the manufacturing process can change and/or improve the critical process parameter candidates.

### Software Implementation Example

The functions of the critical process parameter analysis device 1 (hereinafter, referred to as a "device") can be realized by a program for causing a computer to function as the device, the program causing the computer to function as the control blocks (particularly, the sections included in the control section 10) of the device.

In this case, the device includes, as hardware for executing the program, a computer that includes at least one control device (e.g., a processor) and at least one storage device (e.g., a memory). By the control device executing the program with use of the storage device, the functions described in the foregoing embodiments are realized.

The program may be recorded in one or more non-transitory computer-readable storage media. The one or more storage media may or may not be included in the device. In the latter case, the program may be supplied to or made available to the device via any wired or wireless transmission medium.

Alternatively, a part or all of the functions of the control blocks can be realized by a logic circuit. For example, the present invention encompasses, in its scope, an integrated circuit in which a logic circuit that functions as each of the control blocks is formed. Alternatively, the functions of the control blocks can be realized, for example, by a quantum computer.

The present invention is not limited to the foregoing embodiments, but can be altered by a skilled person in the art within the scope of the claims. The present invention also encompasses, in its technical scope, any embodiment derived by combining technical means disclosed in differing embodiments.

Aspects of the present invention can also be expressed as follows:
A critical process parameter analysis method in accordance with a first aspect of the present invention includes: a variation evaluation step of evaluating, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and a critical process parameter candidate specifying step of specifying a critical process parameter candidate from among the motion parameters on a basis of a result of evaluating, for the each of the plurality of motions, the degree of the variation in the system input and the degree of the variation in the system output.

A critical process parameter analysis method in accordance with a second aspect of the present invention may be configured such that, in the first aspect, the variation in the system input is variation in a motion output which is actually output with respect to the subject system.

A critical process parameter analysis method in accordance with a third aspect of the present invention may be configured such that, in the second aspect, the variation evaluation step evaluates, as the degree of the variation in the system input, a control difficulty which is based on a degree of a difference between (a) a control target value which is set for the corresponding one of the motion parameters in execution of the each of the plurality of motions and (b) an actual control state of the motion output.

A critical process parameter analysis method in accordance with a fourth aspect of the present invention may be configured such that, in the third aspect, the variation evaluation step calculates, for each of the motion parameters, a control difficulty index indicative of the control difficulty.

A critical process parameter analysis method in accordance with a fifth aspect of the present invention may be configured such that, in the fourth aspect, the control difficulty index is an index indicative of a difference between (a) a scale of a range of the control target value and (b) a scale of a controllable range in execution of the each of the plurality of motions.

A critical process parameter analysis method in accordance with a sixth aspect of the present invention may be configured such that, in any one of the first to fifth aspects, the variation in the system output is variation in a response output from the subject system.

A critical process parameter analysis method in accordance with a seventh aspect of the present invention may be configured such that, in any one of the first to sixth aspects, for each of the plurality of motions, a response variability which is based on how much extent the each of the plurality of motions affects an important index reflecting an important quality characteristic of the product is evaluated as the degree of the variation in the system output.

A critical process parameter analysis method in accordance with an eighth aspect of the present invention may be configured such that, in the seventh aspect, the variation evaluation step calculates, for each of the plurality of motions, a response variability index indicative of the response variability.

A critical process parameter analysis method in accordance with a ninth aspect of the present invention may be configured such that, in the eighth aspect, the response variability index is an index indicative of a scale of a difference between the motion output and the response output from the subject system in response to an input with respect to the subject system.

A critical process parameter analysis method in accordance with a tenth aspect of the present invention may be configured such that, in the eighth or ninth aspect, the critical process parameter candidate specifying step specifies the critical process parameter candidate on a basis of a variability index calculated by obtaining a linear sum of (a) the control difficulty index indicative of the control difficulty and (b) the response variability index indicative of the response variability while assigning a given weight.

A critical process parameter analysis method in accordance with an eleventh aspect of the present invention may be configured such that, in any one of the first to tenth aspects, each of the plurality of motions is a motion that a given device is caused to execute in order to execute the partial process.

A critical process parameter analysis method in accordance with a twelfth aspect of the present invention may be configured such that, in any one of the first to eleventh aspects, the product is a cell preparation; and the manufacturing process includes a cell culturing process.

A critical process parameter analysis method in accordance with a thirteenth aspect of the present invention may be configured such that, in the twelfth aspect, the important index includes a viability of cells.

A critical process parameter analysis method in accordance with a fourteenth aspect of the present invention may be configured such that, in any one of the first to eleventh aspects, the product is a chemical product; and the manufacturing process includes a polymerization reaction process.

A critical process parameter analysis device in accordance with a fifteenth aspect of the present invention includes: a variation evaluation section that evaluates, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and a critical process parameter candidate specifying section that specifies a critical process parameter candidate from among the motion parameters on a basis of a result of evaluating, for the each of the plurality of motions, the degree of the variation in the system input and the degree of the variation in the system output.

A critical process parameter analysis program in accordance with a sixteenth aspect of the present invention is a critical process parameter analysis program for causing a computer to function as a critical process parameter analysis device recited in the fifteenth aspect, the critical process parameter analysis program causing the computer to function as the variation evaluation section and the critical process parameter candidate specifying section.

A training method in accordance with a seventeenth aspect of the present invention is a training method of product manufacturing designing involving use of an instruction manual including a critical process parameter analysis method in accordance with any one of the first to fourteenth aspects.

### Reference Signs List

1: critical process parameter analysis device
5: information processing terminal
9: network
10: control section
12: variation evaluation section
13: critical process parameter candidate specifying section
S1: motion specifying step
S2: control difficulty evaluation step (variation evaluation step)
S3: response variability evaluation step (variation evaluation step)
S4: critical process parameter candidate specifying step

## Claims

1. A critical process parameter analysis method comprising:
a variation evaluation step of evaluating, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and
a critical process parameter candidate specifying step of specifying a critical process parameter candidate from among the motion parameters on a basis of a result of evaluating, for the each of the plurality of motions, the degree of the variation in the system input and the degree of the variation in the system output.

2. The critical process parameter analysis method according to claim 1, wherein:
the variation in the system input is variation in a motion output which is actually output with respect to the subject system.

3. The critical process parameter analysis method according to claim 2, wherein:
the variation evaluation step evaluates, as the degree of the variation in the system input, a control difficulty which is based on a degree of a difference between (a) a control target value which is set for the corresponding one of the motion parameters in execution of the each of the plurality of motions and (b) an actual control state of the motion output.

4. The critical process parameter analysis method according to claim 3, wherein:
the variation evaluation step calculates, for each of the motion parameters, a control difficulty index indicative of the control difficulty.

5. The critical process parameter analysis method according to claim 4, wherein:
the control difficulty index is an index indicative of a difference between (a) a scale of a range of the control target value and (b) a scale of a controllable range in execution of the each of the plurality of motions.

6. The critical process parameter analysis method according to claim 1, wherein:
the variation in the system output is variation in a response output from the subject system.

7. The critical process parameter analysis method according to claim 5, wherein:
for each of the plurality of motions, a response variability which is based on how much extent the each of the plurality of motions affects an important index reflecting an important quality characteristic of the product is evaluated as the degree of the variation in the system output.

8. The critical process parameter analysis method according to claim 7, wherein:
the variation evaluation step calculates, for each of the plurality of motions, a response variability index indicative of the response variability.

9. The critical process parameter analysis method according to claim 8, wherein:
the response variability index is an index indicative of a scale of a difference between the motion output and the response output from the subject system in response to an input with respect to the subject system.

10. The critical process parameter analysis method according to claim 8, wherein:
the critical process parameter candidate specifying step specifies the critical process parameter candidate on a basis of a variability index calculated by obtaining a linear sum of (a) the control difficulty index indicative of the control difficulty and (b) the response variability index indicative of the response variability while assigning a given weight.

11. The critical process parameter analysis method according to any one of claims 1 to 10, wherein:
each of the plurality of motions is a motion that a given device is caused to execute.

12. The critical process parameter analysis method according to any one of claims 1 to 10, wherein:
the product is a cell preparation; and
the manufacturing process includes a cell culturing process.

13. The critical process parameter analysis method according to claim 7, wherein:
the product is a cell preparation;
the manufacturing process includes a cell culturing process; and
the important index includes a viability of cells.

14. The critical process parameter analysis method according to any one of claims 1 to 10, wherein:
the product is a chemical product; and
the manufacturing process includes a polymerization reaction process.

15. A critical process parameter analysis device comprising:
a variation evaluation section that evaluates, for each of a plurality of motions included in a manufacturing process of a product, (a) a degree of variation in a system input which is input into a subject system from an outside of the subject system in accordance with a corresponding one of motion parameters which are set to execute the respective plurality of motions and (b) a degree of variation in a system output which is output from the subject system in response to the each of the plurality of motions, the subject system being a subject of the plurality of motions and including a response field exhibiting a response to each of the plurality of motions; and
a critical process parameter candidate specifying section that specifies a critical process parameter candidate from among the motion parameters on a basis of a result of evaluating, for the each of the plurality of motions, the degree of the variation in the system input and the degree of the variation in the system output.

16. A critical process parameter analysis program for causing a computer to function as a critical process parameter analysis device recited in claim 15, the critical process parameter analysis program causing the computer to function as the variation evaluation section and the critical process parameter candidate specifying section.
